# EUROPÄISCHE PATENTANMELDUNG

(11) **EP 2 910 907 A1**
(43) Veröffentlichungstag der Anmeldung: **26.08.2015**
(21) Anmeldenummer: 15000316.8
(22) Anmeldetag: 04.02.2015
(51) Int. Cl.: G01F 1/74, G01F 1/716

(54) **Durchflussmessgerät mit einer ein tomographisches Messprinzip umsetzenden Messvorrichtung**

(30) Priorität: 20.02.2014 DE 102014002224; 10.07.2014 DE 102014010238
(71) Anmelder: Krohne AG, 4019 Basel (CH)
(72) Erfinder: Hogendoorn, Cornelis Johannes, 4211 BG Spijk (NL); Tromp, Rutger Reinout, 3311 EM Dordrecht (NL); Zoeteweij, Marco Leendert, 3344 EP Hendrik-Ido-Ambach (NL); Boursché, Olaf Jean, 3319 PH Dordrecht (NL)
(74) Vertreter: Gesthuysen Patent- und Rechtsanwälte

(57) **Zusammenfassung**

Beschrieben und dargestellt ist ein Durchflussmessgerät (1) zur Bestimmung des Durchflusses eines durch ein Messrohr (2) strömenden mehrphasigen Mediums. Das dargestellte Durchflussmessgerät (1) weist eine ein tomographisches Messprinzip umsetzende Messvorrichtung (3) und eine zweite Messvorrichtung (4) auf. Mindestens eine der beiden Messvorrichtungen (3) bzw. (4) setzt ein auf Kernspinresonanz basierendes Messprinzip um.

Im dargestellten Ausführungsbeispiel ist die zweite Messvorrichtung (4) durch einen Magnetresonanztomographen (7) realisiert. Die erste Messvorrichtung (3) ist davon abweichend realisiert, nämlich durch eine das Messprinzip der Vormagnetisierungskontrastmessung umsetzende Messvorrichtung, die eine Vormagnetisierungsstrecke (8) mit einem konstanten Magnetfeld aufweist. Das Magnetfeld weist mindestens eine Komponente senkrecht zur Durchflussrichtung des mehrphasigen Mediums auf und wird durch Magnetfelderzeugungselemente (9) erzeugt, die um das Messrohr (2) angeordnet sind. Im übrigen gehört zu der Messvorrichtung (3) eine Baueinheit (10) zur Anregung der Kernspins durch einen RF-Anregungsimpuls oder eine RF-Anregungspulssequenz.

## Beschreibung

Die Erfindung betrifft ein Durchflussmessgerät zur Bestimmung des Durchflusses eines durch ein Messrohr strömenden mehrphasigen Mediums, mit einer ein tomographisches Messprinzip umsetzenden Messvorrichtung. Die Erfindung betrifft auch ein Verfahren zum Betreiben eines solchen Durchflussmessgeräts.

Die Atomkerne der Elemente, die einen Kernspin besitzen, besitzen auch ein durch den Kernspin hervorgerufenes magnetisches Moment. Der Kernspin kann als ein durch einen Vektor beschreibbarer Drehimpuls aufgefasst werden, und entsprechend kann auch das magnetische Moment durch einen Vektor beschrieben werden, der parallel zum Vektor des Drehimpulses ist. Der Vektor des magnetischen Moments eines Atomkerns richtet sich bei Anwesenheit eines makroskopischen Magnetfelds parallel zu dem Vektor des makroskopischen Magnetfelds an der Stelle des Atomkerns aus. Dabei präzediert der Vektor des magnetischen Moments des Atomkerns um den Vektor des makroskopischen Magnetfelds an der Stelle des Atomkerns. Die Frequenz der Präzession wird als Lamorfrequenz *ω_{L}* bezeichnet und ist dem Betrag der Magnetfeldstärke *B* proportional. Die Lamorfrequenz wird gemäß ω_{L} = γ · B berechnet. Darin ist *γ* das gyromagnetische Verhältnis, welches für Wasserstoffatome maximal ist. Das gyromagnetische Verhältnis gibt den Proportionalitätsfaktor zwischen dem Drehimpuls bzw. dem Spin eines Teilchens und dem dazugehörigen magnetischen Moment an.

Mess- und Analyseverfahren, die die Eigenschaft der Präzession von Atomkernen mit einem magnetischen Moment bei Anwesenheit eines makroskopischen Magnetfelds ausnutzen, werden als kernmagnetische Resonanz- Mess- oder -Analyseverfahren bezeichnet. Der englische Begriff für kernmagnetische Resonanz ist *Nuclear Magnetic Resonance* (NMR).

Ein wichtiger Vertreter der Messprinzipien ist die Magnetresonanztomographie (MR, oder aus dem Englischen *Magnetic Resonance Imaging,* MRI). Für gewöhnlich werden die von den präzedierenden Atomkernen unter verschiedenen Randbedingungen in einer Sensorspule induzierten elektrischen Signale als Ausgangsgröße für die Mess- und Analyseverfahren verwendet.

Ein Beispiel für Messgeräte, die die kernmagnetische Resonanz ausnutzen, sind kernmagnetische Durchflussmessgeräte, die den Durchfluss eines durch ein Messrohr strömenden mehrphasigen Mediums messen und das Medium analysieren.

Voraussetzung für eine Analyse unter Ausnutzung kernmagnetischer Resonanz ist, dass die zu analysierenden Phasen des Mediums zu unterscheidbaren kernmagnetischen Resonanzen angeregt werden können. Die Analyse kann die Strömungsgeschwindigkeiten der einzelnen Phasen des Mediums und die relativen Anteile der einzelnen Phasen am mehrphasigen Medium umfassen. Kernmagnetische Durchflussmessgeräte können zum Beispiel zur Analyse des aus Ölquellen geförderten mehrphasigen Mediums eingesetzt werden. Das Medium besteht dann im Wesentlichen aus den Phasen Rohöl, Erdgas und Salzwasser, wobei alle Phasen Wasserstoffatomkerne enthalten.

Die Analyse des aus Ölquellen geförderten Mediums kann auch mit sogenannten Testseparatoren erfolgen. Diese zweigen einen kleinen Teil des geförderten Mediums ab, trennen die einzelnen Phasen des Mediums voneinander und bestimmen die Anteile der einzelnen Phasen an dem Medium. Jedoch sind Testseparatoren nicht im Stande, Rohölanteile kleiner als 5 % zuverlässig zu messen. Da der Rohölanteil einer jeden Quelle stetig sinkt und der Rohölanteil vieler Quellen bereits kleiner als 5 % ist, ist es derzeit nicht möglich, diese Quellen unter Verwendung von Testseparatoren wirtschaftlich auszubeuten. Um auch Quellen mit einem sehr geringen Rohölanteil weiterhin ausbeuten zu können, sind dementsprechend genaue Durchflussmessgeräte erforderlich.

Für gewöhnlich werden die von den präzedierenden Atomkernen nach Anregungen in einer Sensorspule induzierten elektrischen Signale als Ausgangsgröße für die Auswertung verwendet. Voraussetzung für die Messung eines mehrphasigen Mediums ist, wie bereits ausgeführt, dass die einzelnen Phasen des Mediums zu unterscheidbaren kernmagnetischen Resonanzen angeregt werden können. Die Größe der von den präzedierenden Atomkernen einer Phase des Mediums in der Sensorspule induzierten elektrischen Signale ist abhängig von der Anzahl der präzedierenden Atomkerne pro Volumenelement in dieser Phase, demnach also abhängig von der Dichte der Phase, aber auch von der Beeinflussungsdauer der präzedierenden Atomkerne im beeinflussenden gesteuerten Magnetfeld. Folglich ist die Größe der induzierten elektrischen Signale bei den flüssigen Phasen größer als bei den gasförmigen Phasen.

Die für die Magnetresonanztomographie notwendige Ortsinformation wird zum Beispiel durch ein Gradientenfeld auf die Probe aufgebracht. Da die Lamorfrequenz der Kernspins proportional zu der Magnetfeldstärke ist, entsteht durch das Gradientenfeld eine ortsabhängige Verteilung verschiedener Lamorfrequenzen der Kernspins und damit eine Ortsabhängigkeit der von den Atomkernen induzierten elektrischen Signale.

Wie bereits oben aufgeführt, ist das MR-Signal abhängig von der Dichte des Mediums. Bei einem Vergleich der durchschnittlichen Werte der Signalamplituden pro Kubikmeter von Gas, Öl und Wasser lässt sich feststellen, dass sich das Signal von Gas deutlich von dem von Öl und von Wasser unterscheidet, jedoch zwischen den Signalen von Öl und von Wasser kaum ein Unterschied besteht. Die Stärke der Signale kann durch den sogenannten Wasserstoffindex *HI* ausgedrückt werden. Der Wasserstoffindex HI beschreibt den relativen Anteil von Wasserstoffatomen eines Mediums im Vergleich zu Wasser. Demnach ist der Wasserstoffindex von Wasser *HI_{wasser}* = 1. Für die Indizes von Öl und Gas gilt *HI_{Öl}* = 0.9 - 1.1 und *HI_{Gas} =* 0 - 0.2. Mit Hilfe der MR-Signale lässt sich demnach gut zwischen Gas auf der einen und Flüssigkeit (bestehend aus Wasser und Öl) auf der anderen Seite unterscheiden. Eine Unterscheidung von Wasser und Öl hingegen erweist sich als schwierig bzw. sehr aufwendig, da sich die Amplituden der MR-Signale kaum unterscheiden.

Wie bereits aufgeführt, basieren kernmagnetische Mess- und Analyseverfahren auf dem Effekt, dass sich die magnetischen Momente des Kerns entlang der Feldlinien eines extern angelegten Magnetfeldes ausrichten. Dies führt zu einer Bulkmagnetisierung des Mediums. Die Rate, mit der sich diese Magnetisierung einstellt, wird durch die sogenannte Spin-Gitter-Relaxationszeit T₁ bestimmt und weist einen exponentiellen Verlauf auf.

Eine weitere, für kernmagnetische Mess- und Analyseverfahren typische Messgröße ist die Spin-Spin-Relaxationszeit T₂. Diese Zeit ist ein Maß für Inhomogenitäten in dem einen einzelnen Spin umgebenden Magnetfeld.

Die Mechanismen, die die Werte für T₁ und T₂ bestimmen, sind abhängig von der Molekulardynamik der Testprobe. Die Molekulardynamik ist wiederum abhängig von der Größe der Moleküle und ebenso von den intermolekularen Abständen. Diese sind für jedes Medium unterschiedlich. Demnach weisen verschiedene Medien ebenfalls unterschiedliche Werte für T₁ und T₂ auf.

Eine aus dem Stand der Technik bekannte Messmethode zur Charakterisierung einzelner Phasen eines mehrphasigen Mediums ist durch das Messverfahren der Vormagnetisierungskontrastmessung gegeben. Dieses Messverfahren beruht auf dem Unterschied in der T₁-Zeit für verschiedene Phasen eines mehrphasigen Mediums und eignet sich in besonderer Weise zur Bestimmung des Ölanteils und des Wasseranteils sowie dem relativen Verhältnis des Ölanteils zum Wasseranteil in einer Probe.

Das mehrphasige Medium fließt durch eine mit einem konstanten Magnetfeld durchsetzte Strecke. Hierbei weist das Magnetfeld mindestens eine Komponente senkrecht zur Fließrichtung des Mediums auf. Da die Ausrichtung der magnetischen Momente im Magnetfeld abhängig ist von der jeweiligen Phase des Mediums, kommt es bei gleicher Einwirkdauer des Magnetfeldes zu unterschiedlicher Ausbildung der Magnetisierung in den einzelnen Phasen. Die Einwirkdauer des Magnetfeldes wird durch die Länge der mit dem konstanten Magnetfeld durchsetzten Strecke und der Fließgeschwindigkeit des Mediums bestimmt.

Im Allgemeinen ist die longitudinale Relaxationszeit T₁ für Öl deutlich kleiner als die für Wasser. Demnach stellt sich die Magnetisierung von Öl parallel zu dem äußeren Magnetfeld schneller ein als die von Wasser. Durch Variation der Länge der Vormagnetisierungsstrecke sind also die Signale von Öl und Wasser jeweils zu einem unterschiedlichen Level ausgebildet, so dass sich aus den von der Vormagnetisierungsstrecke abhängigen Öl-Wasser-Signalverhältnissen auf das Verhältnis von Ölanteil zu Wasseranteil in dem Medium schließen lässt. Der starke Kontrast zwischen Ölsignal und Wassersignal in Abhängigkeit der Vormagnetisierungsstrecke bietet eine gute Möglichkeit, das Öl-Wasser-Verhältnis (OWR) des Mediums zu bestimmen.

Da das Signal des Gasanteils sehr schwach ist, ist die Methode zum einen unabhängig von dem Gasanteil, auf der anderen Seite jedoch eignet sie sich auch nicht, diesen zu bestimmen, so dass nicht alle drei Phasen des Mediums durch die Messmethode der Vormagnetisierungskontrastmessung charakterisiert werden können.

Ein anderes Messverfahren, welches ebenfalls oftmals Anwendung in der Durchflussmesstechnik findet und nicht auf Kernspinresonanz beruht, ist durch die elektrische Kapazitätstomographie (aus dem Englischen *electrical capacitance tomography,* ECT) gegeben.

Elektrische Kapazitätstomographie ist eine aus dem Stand der Technik bekannte Methode, um mehrphasige Medien zu messen und zu charakterisieren. Sie eignet sich in der Regel für dielektrische Materialien und basiert auf der Tatsache, dass unterschiedliche Materialien unterschiedliche Permittivitäten aufweisen.

Eine typische Messvorrichtung für die elektrische Kapazitätstomographie ist derart ausgestaltet, dass um ein Messrohr herum eine bestimmte Anzahl Elektroden angeordnet ist. Aus dem Stand der Technik bekannte Messvorrichtungen weisen meist eine Anzahl von acht, zwölf oder sechzehn Elektroden auf.

Bei einer Messvorrichtung der in Rede stehenden Art wird eine Anregungsspannung an eine Elektrode angelegt und an allen anderen Elektroden wird die induzierte Spannung / der Strom gemessen, während ihr elektrisches Potential auf Null gehalten wird. Dies wird für alle vorhandenen Elektroden durchgeführt. Am Beispiel einer Messvorrichtung mit acht Elektroden dient in einem ersten Schritt Elektrode 1 als Anregungselektrode und die Elektroden 2 bis 8 als Detektorelektroden. Im nächsten Schritt dient die Elektrode 2 als Anregungselektrode und die Elektroden 3 bis 8 als Detektorelektroden usw.. Bei einer Messvorrichtung mit N Elektroden gibt es also N · (N - 1) / 2 Elektrodenpaarkombinationen und damit N · (N - 1) / 2 Messwerte der Kapazität, aus denen ein Bild konstruiert werden kann. Die Konstruktion geschieht mittels eines Auswertealgorithmus, auf den an dieser Stelle nicht näher eingegangen wird.

Da die Kapazität abhängig von der Permittivität, also der Durchlässigkeit eines Materials für ein elektrisches Feld, des mehrphasigen Mediums zwischen den Elektroden ist, ist es demnach möglich, durch die gemessenen Werte die Verteilung der einzelnen Phasen aufzulösen, da jede Phase des Mediums eine andere Permittivität aufweist.

Die Permittivität für Gas liegt ungefähr bei 1, εᵣ ≈ 1, die Permittivität von Öl zwischen 2 und 4, εᵣ ≈ 2 - 4, und die Permittivität von Wasser ist größer als 50, εᵣ > 50. Anhand der hier aufgezeigten Werte für die Permittivität der einzelnen Phasen ist bereits festzustellen, dass es sich als sehr schwierig und aufwendig erweist, die gasförmige Phase von der Ölphase zu separieren, da die die beiden Phasen charakterisierenden Werte der Permittivitäten nicht weit auseinanderliegen, nämlich also fast gleich sind. Elektrische Kapazitätstomographie erweist sich aus dem oben Aufgeführten als gute Methode, um den Kohlenwasserstoffanteil eines mehrphasigen Mediums, der sich additiv aus dem Ölanteil und dem Gasanteil zusammensetzt, und den Wasseranteil des Mediums zu bestimmen.

Die oben aufgeführten Messmethoden haben, wie dargelegt, große Vorteile bei der Messung bestimmter Eigenschaften eines mehrphasigen Mediums. Auf der anderen Seite weisen sie jedoch auch die dargestellten Nachteile bzw. Einschränkungen auf, so dass die Bestimmung aller drei Phasen des mehrphasigen Mediums entweder nicht möglich, ungenau oder extrem aufwendig ist.

Aufgabe der vorliegenden Erfindung ist demnach die Angabe eines Durchflussmessgerätes und eines Verfahrens zum Betreiben des erfindungsgemäßen Durchflussmessgerätes, mit dem alle drei Phasen des mehrphasigen Mediums auf einfache Weise zuverlässig bestimmt werden können.

Das erfindungsgemäße Durchflussmessgerät, bei dem die zuvor hergeleitete und aufgezeigte Aufgabe gelöst ist, ist zunächst und im Wesentlichen dadurch gekennzeichnet, dass mindestens eine weitere Messvorrichtung vorgesehen ist und mindestens eine der Messvorrichtungen ein auf Kernspinresonanz basierendes Messprinzip umsetzt. Die zweite Messvorrichtung kann entweder ebenfalls ein tomographisches oder aber ein nicht-tomographisches Messprinzip realisieren.

Ein Vorteil des erfindungsgemäßen Durchflussmessgerätes besteht gegenüber den aus dem Stand der Technik bekannten Durchflussmessgeräten darin, dass es möglich ist, den Durchfluss aller drei Phasen des mehrphasigen Mediums zu bestimmen, ohne die einzelnen Phasen separieren zu müssen. Dadurch wird der nötige Aufwand für die Bestimmung des Durchflusses eines durch ein Messrohr strömenden mehrphasigen Mediums deutlich reduziert.

Unterschiedliche Messverfahren haben unterschiedliche Vor- und Nachteile. Bei der Kombination von zwei Messvorrichtungen in einem Durchflussmessgerät, die unterschiedliche Messprinzipien umsetzen, können die Nachteile des einen Messprinzips durch die Vorteile des anderen Messprinzips zumindest teilweise kompensiert werden, so dass mit den kombinierten Messprinzipien optimierte Messergebnisse erzielt werden können.

Eine bevorzugte Ausführungsform des erfindungsgemäßen Durchflussmessgerätes, bei dem das tomographische Messverfahren durch Magnetresonanztomographie realisiert ist, ist ergänzend dadurch gekennzeichnet, dass eine Messvorrichtung das Messprinzip der elektrischen Kapazitätstomographie umsetzt. Dabei sind die beiden Messvorrichtungen in Strömungsrichtung des Mediums hintereinander um das von dem mehrphasigen Medium durchströmte Messrohr angeordnet. Hierbei kann die erste Messvorrichtung die sein, die das tomographische Messprinzip durch Magnetresonanztomographie umsetzt, während dann die zweite Messvorrichtung die ist, die das Messprinzip der elektrischen Kapazitätstomographie umsetzt. Ohne Weiteres ist aber auch die umgekehrte Reihenfolge der beiden Messvorrichtungen möglich.

Die das tomographische Messprinzip durch Magnetresonanztomographie realisierende Messvorrichtung besteht aus einem Magnetresonanztomographen, der um das Messrohr angeordnet ist. Der Magnetresonanztomograph beinhaltet vorzugsweise zumindest eine Magnetfelderzeugungseinrichtung für ein konstantes Magnetfeld und eine Magnetfelderzeugungseinrichtung für ein Gradientenmagnetfeld, diese kann vorzugsweise durch eine Gradientenspule realisiert sein. Das Gradientenfeld kann dem konstanten Magnetfeld überlagert sein, um so eine Ortsinformation auf die Probe aufzubringen. Zudem beinhaltet der Magnetresonanztomograph vorzugsweise ebenfalls eine Signalspule zur Erzeugung eines RF-Anregungspulses oder einer RF-Pulssequenz zur Anregung der Kernspins sowie eine Detektorspule, um die von den Kernspins erzeugten Messsignale detektieren zu können. Hierbei können die Signalspule und die Detektorspule verschiedene Spulen sein oder durch eine Spule realisiert sein.

Ohne Beschränkung der Allgemeinheit sei die Fließrichtung des Mediums durch das Messrohr als x-Richtung definiert. Bei Einführung eines Koordinatensystems ist die x-Achse also entlang der Längsachse des Messrohres gewählt. Die y-Achse ist definiert als die Achse in der Horizontalen, die z-Achse ist definiert als die Achse in der Vertikalen.

Der Magnetresonanztomograph ist nun so ausgestaltet, dass die für die Tomographie nötige Ortsinformation in unterschiedlichen Richtungen aufgebracht werden kann. Die Ortsinformation kann zum Beispiel durch ein Gradientenfeld unterschiedlicher Gradientenrichtungen generiert werden. Dies ist insbesondere insofern vorteilhaft, als dass die Messung der jeweiligen Beschaffenheit des Mediums angepasst werden kann, wodurch der Messaufwand reduziert wird.

Die Erzeugung eines Gradientenfeldes entlang der z-Richtung eignet sich insbesondere dann, wenn ein Medium vorliegt, bei dem die Flüssigphase und die Gasphase "getrennt" vorliegen, die Flüssigphase also zum Beispiel im unteren Bereich des Rohres fließt und die Gasphase aufgrund der geringeren Dichte im oberen Bereich des Rohres strömt. Für ein solches Medium ist es ausreichend, eine Ortsinformation nur in z-Richtung aufzubringen, um das Medium vollständig zu charakterisieren.

Ist das mehrphasige Medium der Art, dass die Gasphase über den gesamten Querschnitt des Messrohres mit der Flüssigphase vermischt ist, also zum Beispiel in Form von Gasblasen in der Flüssigkeit, reicht eine Ortsinformation in z-Richtung nicht aus, um das gesamte Medium zu charakterisieren. Vielmehr ist es nun notwendig, jeden Punkt entlang des Querschnitts des Messrohres messen zu können. Demzufolge ist es nötig, eine Ortsinformation sowohl in z-Richtung als auch in y-Richtung aufzubringen. Die Realisierung kann auf zwei verschiedene Arten durchgeführt werden. Zum einen kann zunächst ein Gradientenfeld entlang der z-Achse erzeugt werden, G = G_{z} · e_{z}. Im Anschluss daran kann ein Gradientenfeld entlang der y-Achse erzeugt werden, G = G_{y} · e_{y}. So wird in einem ersten Schritt die Ortsinformation entlang der z-Achse generiert und gemessen, und in einem zweiten Schritt die Ortsinformation entlang der y-Achse generiert und gemessen. Eine Kombination der so generierten Messergebnissätze ergibt ein vollständiges Bild. Zum anderen ist es möglich ein Gradientenfeld zu erzeugen, das sowohl einen Gradienten entlang der z-Achse, als auch einen Gradienten entlang der y-Achse aufweist, G = G_{y} · e_{y} + G_{z} · e_{z}. Durch ein derartiges Feld wird die Ortsinformation direkt auf den gesamten Querschnitt des Messrohres aufgebracht.

Es ist nun denkbar, auf verschiedene Weisen die Ortsinformation mit Hilfe des Gradientenfeldes zu kodieren.

Liegt das Gradientenfeld bereits vor der Anregung des Spinsystems durch den Anregungspuls an, präzedieren die Spins also bereits vor der Anregung ortsabhängig mit verschiedenen Lamorfrequenzen, kann durch die Pulsbreite des Anregungspulses ein bestimmter Teil der Spins ausgewählt werden, der angeregt wird. Es liegt also eine selektive Anregung des Spinsystems vor, demnach sendet auch nur der selektiv angeregte Teil ein Messsignal aus.

Ebenfalls ist es denkbar, die Ortsinformation durch eine Phasenverschiebung der Spins zu kodieren. Das Gradientenfeld wird zwischen der Anregung des Spinsystems durch einen Anregungspuls und dem Auslesen der von dem Spinsystem generierten Signale für ein bestimmtes Zeitintervall angelegt. Durch das Gradientenfeld wird die Präzessionsfrequenz ortsabhängig verändert, da, wie bereits aufgeführt, die Lamorfrequenz proportional zur Magnetfeldstärke ist. Wird das Gradientenfeld wieder abgeschaltet, präzedieren die Spins wieder mit ihrer "alten" Frequenz, jedoch hat es eine ortsabhängige Änderung der Phasenlage der angeregten Spins gegeben, man spricht von Phasenkodierung.

Werden die Spins durch einen Anregungspuls angeregt und anschließend ein Gradientenfeld während des Auslesens angelegt ("Auslesegradientenfeld"), führt das dazu, dass die Spins während der Messung Signale mit ortsabhängig unterschiedlichen Frequenzen aussenden. Das gemessene "Frequenzgemisch" kann durch eine Fourier-Transformation dekodiert werden. Eine Kodierung der Ortsinformation ist also auch über die Frequenz möglich, man spricht von Frequenzkodierung.

Der als weitere Messvorrichtung vorgesehene elektrische Kapazitätstomograph kann so ausgeführt sein, wie dies weiter oben allgemein für elektrische Kapatitätstomographen beschrieben ist, so dass es sich erübrigt, hier noch auf Details einzugehen.

Der elektrische Kapazitätstomograph beinhaltet eine Anzahl von Elektroden, die symmetrisch um das Messrohr angeordnet sind. Die Anzahl der Elektroden ist beliebig wählbar. Die Elektroden werden vorzugsweise von außen an dem Messrohr befestigt. Dadurch wird gewährleistet, dass der Fluss des Mediums nicht durch die Elektroden gestört und beeinflusst wird. Durch eine Messeinheit wird an eine der Elektroden eine Anregungsspannung angelegt und das Messsignal der übrigen Elektroden detektiert. Dieser Vorgang wird wie oben beschrieben für alle Elektroden wiederholt und durch einen Algorithmus die Verteilung der Permittivitäten in der Probe rekonstruiert.

Eine andere bevorzugte Ausführungsform des erfindungsgemäßen Durchflussmessgeräts, bei dem das tomographische Messprinzip durch elektrische Kapazitätstomographie realisiert ist, ist ergänzend dadurch gekennzeichnet, dass eine Messvorrichtung das Messprinzip der Vormagnetisierungskontrastmessung umsetzt. Die das Messprinzip der Vormagnetisierungskontrastmessung umsetzende Messvorrichtung beinhaltet eine mit einem konstanten Magnetfeld durchsetzte Vormagnetisierungsstrecke, - wobei das Magnetfeld mindestens eine Komponente senkrecht zum strömenden Medium aufweist, sowie eine Baueinheit, mit der die Kernspins durch einen RF-Anregungspuls oder eine RF-Anregungspulssequenz angeregt werden können, und das von den Kernspins erzeugte Messsignal detektiert werden kann.

Um die Messmethode der Vormagnetisierungskontrastmessung umsetzen zu können, muss die mit dem konstanten Magnetfeld durchsetzte Vormagnetisierungsstrecke in ihrer Länge variabel sein, was unterschiedlich realisiert werden kann.

Eingangs ist ausgeführt, dass die Erfindung auch Verfahren zum Betreiben eines Durchflussmessgeräts zur Bestimmung des Durchflusses eines durch ein Messrohr strömenden mehrphasigen Mediums betrifft, wobei zu dem Durchflussmessgerät eine ein tomographisches Messprinzip umsetzende Messvorrichtung gehört.

Hier gibt es jetzt eine Vielzahl von Möglichkeiten, wobei bei allen Möglichkeiten gemeinsam ist, dass zwei Messprinzipien angewendet werden und mindestens eins der Messprinzipien ein auf Kernspinresonanz basierendes Messprinzip ist.

Wird das tomographische Messprinzip durch Magnetresonanztomographie realisiert, dann kann außerdem entweder das Messverfahren der elektrischen Kapazitätstomographie oder das Messverfahren der Vormagnetisierungskontrastmessung angewendet werden. Wird das tomographische Messprinzip durch elektrische Kapazitätstomographie realisiert, dann kann außerdem das Messverfahren der Vormagnetisierungskontrastmessung angewendet werden.

Wird das tomographische Messprinzip durch Magnetresonanztomographie und außerdem das Messverfahren der elektrischen Kapazitätstomographie angewendet, dann kann im Einzelnen so vorgegangen werden, dass durch Magnetresonanztomographie der Gasanteil α_{G} und der Flüssigkeitsanteil α_{L}, der sich additiv aus dem Wasseranteil α_{W} und dem Ölanteil α_{O} ergibt, also α_{L} = a_{W} + α_{O} gemessen werden, wobei die Ortsinformation durch selektive Anregung und/oder Phasenkodierung und/ oder Frequenzkodierung kodiert wird und ein Gradientenfeld entlang der z-Achse angelegt wird, G = G_{z} · e_{z} oder ein Gradientenfeld entlang der y-Achse angelegt wird, G = G_{y} · e_{y} oder ein Gradientenfeld zunächst entlang der z-Achse und anschließend entlang der y-Achse angelegt wird und die beiden Datensätze kombiniert werden oder ein Gradientenfeld gleichzeitig entlang der z-Achse und der y-Achse angelegt wird, G = G_{y} · e_{y} + G_{z} · e_{z}, dass mittels elektrischer Kapazitätstomographie der Wasseranteil α_{W} und der Kohlenwasserstoffanteil α_{C}, der sich additiv aus dem Ölanteil α_{O} und dem Gasanteil a_{G} gemäß a_{C} = α_{O} + α_{G} ergibt, gemessen werden und der Wasseranteil und der Kohlenwasserstoffanteil aus der Verteilung der Permittivitäten oder aus der Verteilung der Leitfähigkeit des Mediums bestimmt werden und dass mit den gemessenen Werten der Ölanteil α_{O} berechnet wird, indem der mittels elektrischer Kapazitätstomographie gemessene Wasseranteil α_{W} von dem mittels Magnetresonanztomographie gemessenen Flüssigkeitsanteil α_{L} subtrahiert wird, also α_{O} = α_{L,MR} - α_{W,ECT}, oder dass der Ölanteil berechnet wird, indem der mittels Magnetresonanztomographie gemessene Gasanteil a_{G} von dem durch elektrische Kapazitätstomographie gemessenen Kohlenwasserstoffanteil a_{C} subtrahiert wird, also α_{O} = α_{C,MRT} -α_{G,MR}.

Bei dem zuletzt beschriebenen Verfahren empfiehlt es sich des Weiteren, die mittlere Leitfähigkeit des Mediums aus den mittels elektrischer Kapazitätstomographie gemessenen Werten zu bestimmen, die durch die mittlere Leitfähigkeit des Mediums und/oder mindestens einer leitfähigen Phase eines mehrphasigen Mediums bedingte zusätzliche Last in dem RF-Resonator-Kreis des Magnetresonanztomographen zu bestimmen und die zur Anregung der Kernspins in das Medium eingespeiste RF-Leistung derart zu erhöhen, dass der Einfluss der durch die mittlere Leitfähigkeit zusätzlich auftretenden Last auf die Anregung der Kernspins kompensiert wird.

Bei dem zuletzt beschriebenen Verfahren empfiehlt es sich weiter, so vorzugehen, dass mittels elektrischer Kapazitätstomographie eine Leitfähigkeitskarte des Mediums über den Rohrquerschnitt erstellt wird, dass aus der Leitfähigkeitskarte die mittlere Leitfähigkeit des Mediums berechnet wird, und dass zusätzlich mit der Leitfähigkeitskarte die lokalen Abweichungen der Leitfähigkeit von der mittleren Leitfähigkeit des Mediums bestimmt werden, dass die durch die mittlere Leitfähigkeit des Mediums bestimmte zusätzliche Last in dem RF-Resonator-Kreis des Magnetresonanztomographen bestimmt wird und dass zusätzlich die lokalen aufgrund der Abweichungen der lokalen Leitfähigkeiten von der mittleren Leitfähigkeit auftretenden Dämpfungen des RF-Feldes bestimmt werden, und dass die zur Anregung der Kernspins in das Medium eingespeiste RF-Leistung derart erhöht wird, dass der Einfluss der durch die mittlere Leitfähigkeit zusätzlich auftretenden Last auf die Anregung der Kernspins kompensiert wird und zusätzlich lokal RF-Leistung in das Medium eingespeist wird, derart dass der Einfluss der von der mittleren Leitfähigkeit abweichenden lokalen Leitfähigkeiten auf die Anregung der Kernspins kompensiert wird.

Wird bei dem erfindungsgemäßen Verfahren das tomographische Messprinzip durch Magnetresonanztomographie realisiert und außerdem das Messverfahren der Vormagnetisierungskontrastmessung angewendet, ist eine weitere Lehre der Erfindung dadurch gekennzeichnet, dass durch das Messverfahren der Vormagnetisierungskontrastmessung der Ölanteil a_{O} und der Wasseranteil α_{W} gemessen werden, wobei der Vormagnetisierungskontrast durch Variation der Vormagnetisierungsstrecke oder durch Variation der Messpositionen realisiert wird, und dass durch Magnetresonanztomographie der Gasanteil α_{G} gemessen wird, wobei die Ortsinformation durch selektive Anregung und/oder Phasenkodierung und/ oder Frequenzkodierung kodiert wird und ein Gradientenfeld entlang der z-Achse angelegt wird, G = G_{z} · e_{z}, oder ein Gradientenfeld entlang der y-Achse angelegt wird, G = G_{y} · e_{y}, oder ein Gradientenfeld zunächst entlang der z-Achse und anschließend entlang der y-Achse angelegt wird und die beiden Datensätze kombiniert werden, oder ein Gradientenfeld gleichzeitig entlang der z-Achse und der y-Achse angelegt wird, G = G_{y} · e_{y} + G₂ · e_{z},

Eine andere Realisierung des erfindungsgemäßen Verfahrens, bei dem das tomographische Messprinzip durch Magnetresonanztomographie realisiert ist und außerdem das Messverfahren der Vormagnetisierungskontrastmessung angewendet ist, ist dadurch gekennzeichnet, dass durch das Messverfahren der Vormagnetisierungskontrastmessung das Verhältnis von Ölanteil α_{O} zu Wasseranteil a_{W} bestimmt wird (OWR = α_{O} / α_{W}), wobei der Vormagnetisierungskontrast durch Variation der Länge der Vormagnetisierungsstrecke oder durch Variation der Messpositionen realisiert wird, dass durch Magnetresonanztomographie der Flüssigkeitsanteil α_{L} und der Gasanteil α_{G} gemessen werden, wobei die Ortsinformation durch selektive Anregung und/oder Phasenkodierung und/ oder Frequenzkodierung kodiert wird und ein Gradientenfeld entlang der z-Achse angelegt wird, G = G_{z} · e_{z}, oder ein Gradientenfeld entlang der y-Achse angelegt wird, G = G_{y} · e_{y}, oder ein Gradientenfeld zunächst entlang der z-Achse und anschließend entlang der y-Achse angelegt wird und die beiden Datensätze kombiniert werden, oder ein Gradientenfeld gleichzeitig entlang der z-Achse und der y-Achse angelegt wird, G = G_{y} · e_{y} + G_{z} · e_{z}, und dass der Wasseranteil aus dem durch das Messverfahren der Magnetresonanztomographie gemessenen Flussigkeitsanteil α_{L} und dem durch das Messverfahren der Vormagnetisierungskontrastmessung bestimmten Verhältnis von Öl- zu Wasseranteil OWR gemäß α_{W}=α_{L,MR}/ (OWR +1) berechnet wird.

Das erfindungsgemäße Verfahren kann, wie ausgeführt, auch zum Inhalt haben, das tomographische Messprinzip durch elektrische Kapazitätstomographie zu realisieren und außerdem das Messverfahren der Vormagnetisierungskontrastmessung anzuwenden. Dabei kann im Einzelnen so vorgegangen werden, dass durch elektrische Kapazitätstomographie der Wasseranteil α_{W} und der Kohlenwasserstoffanteil α_{C}, der sich additiv aus dem Ölanteil α_{O} und dem Gasanteil α_{G} gemäß α_{C}=α_{O}+α_{G} ergibt, gemessen werden, und der Wasseranteil und der Kohlenwasserstoffanteil aus der Verteilung der Permittivitäten oder aus der Verteilung der Leitfähigkeit des Mediums bestimmt werden, dass durch das Messverfahren der Vormagnetisierungskontrastmessung der Wasseranteil α_{W} und der Ölanteil α_{O} gemessen werden, wobei der Vormagnetisierungskontrast durch Variation der Länge der Vormagnetisierungsstrecke oder durch Variation der Messpositionen realisiert wird, und dass mit den gemessenen Werten der Gasanteil α_{G} berechnet wird, indem der mittels dem Prinzip der Vormagnetisierungskontrastmessung gemessene Ölanteil α_{O} von dem mittels elektrischer Kapazitätstomographie gemessenen Kohlenwasserstoffanteil α_{C} subtrahiert wird, α_{G}= α_{C,ET} - α_{O,VM}.

Es kann aber auch so vorgegangen werden, dass mittels elektrischer Kapazitätstomographie der Wasseranteil α_{W} und der Kohlenwasserstoffanteil α_{C}, der sich additiv aus dem Ölanteil α_{O} und dem Gasanteil α_{G} gemäß α_{C}=α_{O} + α_{G} ergibt, gemessen werden, und der Wasseranteil α_{W} und der Kohlenwasserstoffanteil α_{C} aus der Verteilung der Permittivitäten oder aus der Verteilung der Leitfähigkeit des Mediums bestimmt werden, dass durch das Messverfahren der Vormagnetisierungskontrastmessung das Verhältnis von Ölanteil α_{O} zu Wasseranteil α_{W} (OWR = α_{O}/α_{W}) bestimmt wird, wobei der Vormagnetisierungskontrast durch Variation der Länge der Vormagnetisierungsstrecke oder durch Variation der Messpositionen realisiert wird, und dass mit den gemessenen Werten zunächst der Ölanteil α_{O} berechnet wird, indem der mittels elektrischer Kapazitätstomographie gemessene Wasseranteil α_{W} mit dem mittels dem Prinzip der Vormagnetisierungskontrastmessung bestimmten OWR multipliziert wird, α_{O} = OWR · α_{W},_{ET}, und anschließend der Gasanteil α_{G} bestimmt wird, indem der zuvor bestimmte Ölanteil α_{O} von dem mittels elektrischer Kapazitätstomographie gemessenen Kohlenwasserstoffanteil α_{C} subtrahiert wird, α_{G}=α_{C,ECT} - α_{O}.

Bei der konkreten Realisierung des Verfahrens, bei dem das tomographische Messprinzip durch elektrische Kapazitätstomographie realisiert ist und außerdem das Messverfahren der Vormagnetisierungskontrastmessung angewendet wird, die zuvor im Einzelnen beschrieben worden ist, kann ergänzend so vorgegangen werden, dass die mittlere Leitfähigkeit des Mediums aus den mittels elektrischer Kapazitätstomographie gemessenen Werten bestimmt wird, dass die durch die mittlere Leitfähigkeit des Mediums und/oder mindestens einer leitfähigen Phase eines mehrphasigen Mediums bedingte zusätzliche Last in dem RF-Resonator-Kreis des Magnetresonanzmessgerätes bestimmt wird und dass die zur Anregung der Kernspins in das Medium eingespeiste RF-Leistung derart erhöht wird, dass der Einfluss der durch die mittlere Leitfähigkeit zusätzlich auftretenden Last auf die Anregung der Kernspins kompensiert wird.

Dies kann im Einzelnen ergänzend dadurch realisiert werden, dass mittels elektrischer Kapazitätstomographie eine Leitfähigkeitskarte des Mediums über den Rohrquerschnitt erstellt wird, dass aus der Leitfähigkeitskarte die mittlere Leitfähigkeit des Mediums berechnet wird, und dass zusätzlich mit der Leitfähigkeitskarte die lokalen Abweichungen der Leitfähigkeit von der mittleren Leitfähigkeit des Mediums bestimmt werden,
dass die durch die mittlere Leitfähigkeit des Mediums bestimmte zusätzliche Last in dem RF-Resonator-Kreis des Magnetresonanzmessgerätes bestimmt wird und dass zusätzlich die lokalen aufgrund der Abweichungen der lokalen Leitfähigkeiten von der mittleren Leitfähigkeit auftretenden Dämpfungen des RF-Feldes bestimmt werden und dass die zur Anregung der Kernspins in das Medium eingespeiste RF-Leistung derart erhöht wird, dass der Einfluss der durch die mittlere Leitfähigkeit zusätzlich auftretenden Last auf die Anregung der Kernspins kompensiert wird und zusätzlich lokal RF-Leistung in das Medium eingespeist wird, derart dass der Einfluss der von der mittleren Leitfähigkeit abweichenden lokalen Leitfähigkeiten auf die Anregung der Kernspins kompensiert wird.

Schließlich kann das erfindungsgemäße Verfahren, wie es zuvor im Einzelnen beschrieben worden ist, auch dazu verwendet werden, um aus der Leitfähigkeit des Mediums und/oder mindestens einer leitfähigen Phase des mehrphasigen Mediums den Salzgehalt in dem Medium und/oder in mindestens einer leitfähigen Phase des mehrphasigen Mediums zu bestimmen.

Im Einzelnen gibt es verschiedene Möglichkeiten zur Ausgestaltung und Weiterbildung des erfindungsgemäßen Durchflussmessgeräts. Dazu wird verwiesen auf die dem Patentanspruch 1 nachgeordneten Patentansprüche und auf die Beschreibung in Verbindung mit der Zeichnung. In der Zeichnung zeigen
- Fig. 1: ein erstes Ausführungsbeispiel eines erfindungsgemäßen Durchflussmessgeräts, bei dem das Messprinzip der Magnetresonanztomographie und das Messprinzip der elektrischen Kapazitätstomographie umgesetzt ist,
- Fig. 2: ein zweites Ausführungsbeispiel eines erfindungsgemäßen Durchflussmessgeräts, bei dem das Messprinzip der elektrischen Kapazitätstomographie und das Messprinzip der Vormagnetisierungskontrastmessung umgesetzt ist, und
- Fig. 3: ein drittes Ausführungsbeispiels eines erfindungsgemäßen Durchflussmessgeräts, bei dem das Messprinzip der Magnetresonanztomographie und das Messprinzip der Vormagnetisierungskontrastmessung umgesetzt ist.

Alle Figuren zeigen jeweils ein Durchflussmessgerät 1 zur Bestimmung des Durchflusses eines durch ein Messrohr 2 strömenden mehrphasigen Mediums. Allen in den Figuren dargestellten Durchflussmessgeräten 1 ist auch gemeinsam, dass sie zunächst eine ein tomographisches Messprinzip umsetzende erste Messvorrichtung 3 und eine zweite Messvorrichtung 4 aufweisen. Mindestens eine der Messvorrichtungen 3 und 4 setzt ein auf Kernspinresonanz basierendes Messprinzip um.

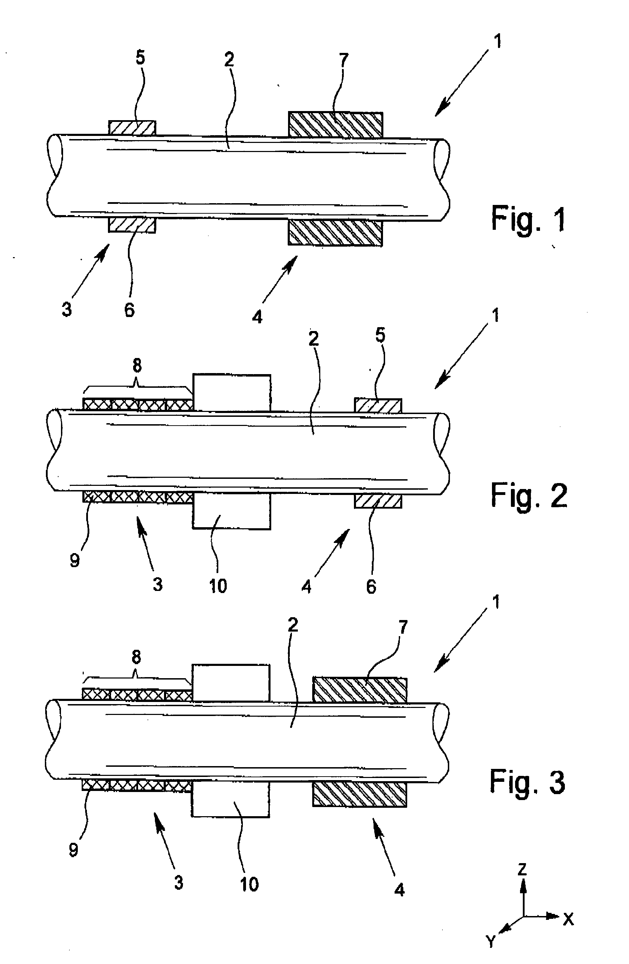

Bei dem in Fig. 1 dargestellten Ausführungsbeispiel des erfindungsgemäßen Durchflussmessgerätes 1 ist die erste Messvorrichtung 3 durch einen elektrischen Kapazitätstomographen 5 realisiert. Dieser elektrische Kapazitätstomograph 5 hat eine Anzahl von Elektroden 6, die symmetrisch um das Messrohr 2 angeordnet sind. Der elektrische Kapatitätstomograph 5 ist so ausgestaltet, dass die Elektroden 6 von Außen an dem Messrohr 2 befestigbar sind. Dadurch ist garantiert, dass das Strömen des mehrphasigen Mediums durch das Messrohr 2 nicht durch die Elektroden 6 behindert oder gestört wird. Die an den Elektroden 6 entstehenden Messsignale werden in einer nicht dargestellten Auswerteeinheit ausgewertet und durch einen geeigneten Algorithmus zu einer zweidimensionalen Permitivitätsverteilungskarte des Querschnitts des Messrohrs 2 konstruiert.

Die zweite Messvorrichtung 4 des in der Fig. 1 dargestellten Durchflussmessgerätes 1 ist durch einen Magnetresonanztomographen 7 realisiert, der ebenfalls um das Messrohr 2 angeordnet ist. Der Magnetresonanztomograph 7 beinhaltet eine im Einzelnen nicht dargestellte Magnetfelderzeugungseinrichtung zur Erzeugung eines konstanten Magnetfeldes und eine ebenfalls im Einzelnen nicht dargestellte Magnetfelderzeugungseinrichtung für ein Gradientenmagnetfeld G, das dem konstanten Magnetfeld überlagert werden kann, eine wiederum nicht dargestellte Anregungsspule zur Erzeugung eines RF-Anregungspulses oder einer RF-Anregungspulssequenz und eine erneut nicht dargestellte Detektorspule, mit der das von den Kernspins erzeugte Messsignal detektiert werden kann. Die Anregungsspule und die Detektorspule können durch eine Spule realisiert sein. Mit dem Magnetresonanztomographen 7 ist es möglich, ein Gradientenfeld G entlang der z-Richtung, G = G_{z} · e_{z}, und/oder ein Gradientenfeld G entlang der y-Richtung, G = G_{y} · e_{y}, zu erzeugen. Dies kann sowohl zeitgleich, G = G_{y} · e_{y} + G_{z} · e_{z}, als auch zeitlich nacheinander erfolgen. Die Definitionen der x-, y- und z-Richtung kann der Zeichnung, unterhalb der Fig. 3, entnommen werden.

Für das Ausführungsbeispiel nach Fig. 2 gilt, dass die erste Messvorrichtung 3 durch eine das Messprinzip der Vormagnetisierungskontrastmessung umsetzende Messvorrichtung realisiert ist, die eine Vormagnetisierungsstrecke 8 mit einem konstanten Magnetfeld aufweist. Das Magnetfeld weist mindestens eine Komponente senkrecht zur Durchflussrichtung des mehrphasigen Mediums auf und wird durch Magnetfelderzeugungselemente 9 erzeugt, die um das Messrohr 2 angeordnet sind. Die von dem Magnetfeld durchsetzte Strecke ist abhängig von der Anzahl der Magnetfelderzeugungselemente 9 und der Richtung der erzeugten Magnetfelder zueinander.

Im Übrigen gehört zu der ersten Messvorrichtung 3 eine Baueinheit 10 zur Anregung der Kernspins durch einen RF-Anregungspuls oder eine RF-Anregungspulssequenz und zur Messung des von den Kernspins erzeugten Messsignals.

Die zweite Messvorrichtung 4 ist in dem in der Fig. 2 dargestellten Ausführungsbeispiel eines erfindungsgemäßen Durchflussmessgeräts durch einen elektrischen Kapazitätstomographen 5 realisiert. Dieser Kapazitätstomograph 5 kann genau so realisiert sein und mit ihm kann genau das gewonnen werden, was weiter oben in Verbindung mit dem zum Ausführungsbeispiel nach Fig. 1 gehörenden Kapazitätstomographen 5 gesagt ist.

Für das in Fig. 3 dargestellte Ausführungsbeispiel eines erfindungsgemäßen Durchflussmessgerätes 1 gilt, wie bereits weiter oben ausgeführt, dass es eine erste Messvorrichtung 3 und eine zweite Messvorrichtung 4 aufweist. Dabei setzt die erste Messvorrichtung 3 das Messprinzip der Vormagnetisierungskontrastmessung um und beinhaltet eine Vormagnetisierungsstrecke 8, die mit einem konstanten Magnetfeld durchsetzt ist. Auch hier wird das Magnetfeld durch eine Anzahl von Magnetfelderzeugungselementen 9 erzeugt, die um das Messrohr 2 angeordnet sind, und weist mindestens eine Komponente senkrecht zur Durchflussrichtung des mehrphasigen Mediums auf. Auch hier beinhaltet die Messvorrichtung 3 eine Baueinheit 10 zur Anregung des Kernspins mit einem RF-Anregungspuls oder einer RF-Anregungspulssequenz und zur Messung der von den Kernspins erzeugten Messsignale. Durch die Anzahl der Magnetfelderzeugungselemente 9 und/oder die Richtung der durch die Magnetfelderzeugungselemente 9 erzeugten Magnetfelder zueinander wird die mit dem effektiven Magnetfeld durchsetzte Vormagnetisierungsstrecke 8 definiert und variiert.

Für das in Fig. 3 schematisch dargestellte Ausführungsbeispiel eines erfindungsgemäßen Durchflussmessgerätes 1 gilt weiter, dass das tomographische Messprinzip durch Magnetresonanztomographie realisiert ist. Hierzu gehört also ein Magnetresonanztomograph 7. Dieser Magnetresonanztomograph 7 kann genau so realisiert sein und mit ihm kann genau das gewonnen werden, was weiter oben in Verbindung mit dem zum Ausführungsbeispiel nach Fig. 1 gehörenden Magnetresonanztomographen 7 gesagt ist.

## Patentansprüche

1. Durchflussmessgerät zur Bestimmung des Durchflusses eines durch ein Messrohr strömenden mehrphasigen Mediums, mit einer ein tomographisches Messprinzip umsetzenden Messvorrichtung,
**dadurch gekennzeichnet,**
**dass** mindestens eine weitere Messvorrichtung vorgesehen ist und mindestens eine der Messvorrichtungen ein auf Kernspinresonanz basierendes Messprinzip umsetzt.

2. Durchflussmessgerät nach Anspruch 1, wobei das tomographische Messprinzip durch Magnetresonanztomographie realisiert ist, **dadurch gekennzeichnet, dass** eine Messvorrichtung das Messprinzip der elektrischen Kapazitätstomographie umsetzt.

3. Durchflussmessgerät nach Anspruch 1, wobei das tomographische Messprinzip durch elektrische Kapazitätstomographie realisiert ist, **dadurch gekennzeichnet, dass** eine Messvorrichtung das Messprinzip der Vormagnetisierungskontrastmessung umsetzt.

4. Durchflussmessgerät nach Anspruch 1, wobei das tomographische Messprinzip durch Magnetresonanztomographie realisiert ist, **dadurch gekennzeichnet, dass** eine Messvorrichtung das Messprinzip der Vormagnetisierungskontrastmessung umsetzt.

5. Verfahren zum Betreiben eines Durchflussmessgerätes zur Bestimmung des Durchflusses eines durch ein Messrohr strömenden mehrphasigen Mediums mit einer ein tomographisches Messprinzip umsetzenden Messvorrichtung,
**dadurch gekennzeichnet,**
**dass** zwei Messprinzipien angewendet werden und mindestens eins der Messprinzipien ein auf Kernspinresonanz basierendes Messprinzip ist.

6. Verfahren nach Anspruch 5, wobei das tomographische Messprinzip durch Magnetresonanztomographie realisiert ist, **dadurch gekennzeichnet, dass** außerdem das Messverfahren der elektrischen Kapazitätstomographie angewendet wird,

7. Verfahren nach Anspruch 5, wobei das tomographische Messprinzip durch Magnetresonanztomographie realisiert ist, **dadurch gekennzeichnet, dass** außerdem das Messverfahren der Vormagnetisierungskontrastmessung angewendet wird.

8. Verfahren nach Anspruch 5, wobei das tomographische Messprinzip durch elektrische Kapazitätstomographie realisiert ist, **dadurch gekennzeichnet, dass** außerdem das Messverfahren der Vormagnetisierungskontrastmessung angewendet wird.

9. Verfahren nach Anspruch 6, **dadurch gekennzeichnet,**
**dass** durch Magnetresonanztomographie der Gasanteil α_{G} und der Flüssigkeitsanteil α_{L}, der sich additiv aus dem Wasseranteil α_{W} und dem Ölanteil α_{O} ergibt, also α_{L} = α_{W} + α_{O}, gemessen werden, wobei die Ortsinformation durch selektive Anregung und/oder Phasenkodierung und/ oder Frequenzkodierung kodiert wird und ein Gradientenfeld entlang der z-Achse angelegt wird, G = G_{z} · e_{z} oder ein Gradientenfeld entlang der y-Achse angelegt wird, G = G_{y} · e_{y} oder ein Gradientenfeld zunächst entlang der z-Achse und anschließend entlang der y-Achse angelegt wird und die beiden Datensätze kombiniert werden oder ein Gradientenfeld gleichzeitig entlang der z-Achse und der y-Achse angelegt wird, G = G_{y} · e_{y} + G_{z} · e_{z},
**dass** mittels elektrischer Kapazitätstomographie der Wasseranteil α_{W} und der Kohlenwasserstoffanteil α_{C}, der sich additiv aus dem Ölanteil α_{O} und dem Gasanteil α_{G} gemäß α = α_{O}+α_{G} ergibt, gemessen werden und der Wasseranteil und der Kohlenwasserstoffanteil aus der Verteilung der Permittivitäten oder aus der Verteilung der Leitfähigkeit des Mediums bestimmt werden und dass mit den gemessenen Werten der Ölanteil α_{O} berechnet wird, indem der mittels elektrischer Kapazitätstomographie gemessene Wasseranteil α_{W} von dem mittels Magnetresonanztomographie gemessenen Flüssigkeitsanteil α_{L} subtrahiert wird, also α_{O} = α_{L,MR} - α_{W,ECT}, oder dass der Ölanteil α_{O} berechnet wird, indem der mittels Magnetresonanztomographie gemessene Gasanteil α_{G} von dem durch elektrische Kapazitätstomographie gemessenen Kohlenwasserstoffanteil α_{C} subtrahiert wird, also α_{O} = α_{C,MRT}- α_{G,MR}.

10. Verfahren nach Anspruch 9, **dadurch gekennzeichnet,**
**dass** die mittlere Leitfähigkeit des Mediums aus den mittels elektrischer Kapazitätstomographie gemessenen Werten bestimmt wird,
**dass** die durch die mittlere Leitfähigkeit des Mediums und/oder mindestens einer leitfähigen Phase eines mehrphasigen Mediums bedingte zusätzliche Last in dem RF-Resonator-Kreis des Magnetresonanztomographen bestimmt wird und
**dass** die zur Anregung der Kernspins in das Medium eingespeiste RF-Leistung derart erhöht wird, dass der Einfluss der durch die mittlere Leitfähigkeit zusätzlich auftretenden Last auf die Anregung der Kernspins kompensiert wird.

11. Verfahren nach Anspruch 10, **dadurch gekennzeichnet,**
**dass** mittels elektrischer Kapazitätstomographie eine Leitfähigkeitskarte des Mediums über den Rohrquerschnitt erstellt wird, dass aus der Leitfähigkeitskarte die mittlere Leitfähigkeit des Mediums berechnet wird, und dass zusätzlich mit der Leitfähigkeitskarte die lokalen Abweichungen der Leitfähigkeit von der mittleren Leitfähigkeit des Mediums bestimmt werden,
**dass** die durch die mittlere Leitfähigkeit des Mediums bestimmte zusätzliche Last in dem RF-Resonator-Kreis des Magnetresonanztomographen bestimmt wird und dass zusätzlich die lokalen aufgrund der Abweichungen der lokalen Leitfähigkeiten von der mittleren Leitfähigkeit auftretenden Dämpfungen des RF-Feldes bestimmt werden, und
**dass** die zur Anregung der Kernspins in das Medium eingespeiste RF-Leistung derart erhöht wird, dass der Einfluss der durch die mittlere Leitfähigkeit zusätzlich auftretenden Last auf die Anregung der Kernspins kompensiert wird und zusätzlich lokal RF-Leistung in das Medium eingespeist wird, derart dass der Einfluss der von der mittleren Leitfähigkeit abweichenden lokalen Leitfähigkeiten auf die Anregung der Kernspins kompensiert wird.

12. Verfahren nach Anspruch 7, **dadurch gekennzeichnet,**
**dass** durch das Messverfahren der Vormagnetisierungskontrastmessung der Ölanteil α_{O} und der Wasseranteil α_{W} gemessen werden, wobei der Vormagnetisierungskontrast durch Variation der Vormagnetisierungsstrecke oder durch Variation der Messpositionen realisiert wird, und
**dass** durch Magnetresonanztomographie der Gasanteil α_{G} gemessene wird, wobei die Ortsinformation durch selektive Anregung und/oder Phasenkodierung und/ oder Frequenzkodierung kodiert wird und ein Gradientenfeld entlang der z-Achse angelegt wird, G = G_{z} · e_{z}, oder ein Gradientenfeld entlang der y-Achse angelegt wird, G = G_{y} · e_{y}, oder ein Gradientenfeld zunächst entlang der z-Achse und anschließend entlang der y-Achse angelegt wird und die beiden Datensätze kombiniert werden, oder ein Gradientenfeld gleichzeitig entlang der z-Achse und der y-Achse angelegt wird, G = G_{y} · e_{y} + G_{z} · e_{z}.

13. Verfahren nach Anspruch 7, **dadurch gekennzeichnet,**
**dass** durch das Messverfahren der Vormagnetisierungskontrastmessung das Verhältnis von Ölanteil α_{O} zu Wasseranteil α_{W} bestimmt wird (OWR = α_{O} / α_{W}), wobei der Vormagnetisierungskontrast durch Variation der Länge der Vormagnetisierungsstrecke oder durch Variation der Messpositionen realisiert wird,
**dass** durch Magnetresonanztomographie der Flüssigkeitsanteil α_{L} und der Gasanteil α_{G} gemessen werden, wobei die Ortsinformation durch selektive Anregung und/oder Phasenkodierung und/ oder Frequenzkodierung kodiert wird und ein Gradientenfeld entlang der z-Achse angelegt wird, G = G_{z} · e_{z}, oder ein Gradientenfeld entlang der y-Achse angelegt wird, G = G_{y} · e_{y}, oder ein Gradientenfeld zunächst entlang der z-Achse und anschließend entlang der y-Achse angelegt wird und die beiden Datensätze kombiniert werden, oder ein Gradientenfeld gleichzeitig entlang der z-Achse und der y-Achse angelegt wird, G = G_{y} · e_{y} + G_{z} · e_{z}, und
**dass** der Wasseranteil α_{W} aus dem mittels Magnetresonanztomographie gemessenen Flüssigkeitsanteil α_{L} und dem durch das Messverfahren der Vormagnetisierungskontrastmessung bestimmten Verhältnis von Öl- zu Wasseranteil OWR gemäß α_{W}= α_{L,MR} / (OWR +1) berechnet wird.

14. Verfahren nach Anspruch 8, **dadurch gekennzeichnet,**
**dass** mittels elektrischer Kapazitätstomographie der Wasseranteil α_{W} und der Kohlenwasserstoffanteil α_{C}, der sich additiv aus dem Ölanteil α_{O} und dem Gasanteil α_{G} gemäß α_{C} = α_{O} + α_{G} ergibt, gemessen werden, und der Wasseranteil und der Kohlenwasserstoffanteil aus der Verteilung der Permittivitäten oder aus der Verteilung der Leitfähigkeit des Mediums bestimmt werden,
**dass** durch das Messverfahren der Vormagnetisierungskontrastmessung der Wasseranteil α_{W} und der Ölanteil α_{O} gemessen werden, wobei der Vormagnetisierungskontrast durch Variation der Länge der Vormagnetisierungsstrecke oder durch Variation der Messpositionen realisiert wird, und
**dass** mit den gemessenen Werten der Gasanteil α_{G} berechnet wird, indem der mittels dem Messverfahren der Vormagnetisierungskontrastmessung gemessene Ölanteil α_{O} von dem mittels elektrischer Kapazitätstomographie gemessenen Kohlenwasserstoffanteil α_{C} subtrahiert wird, α_{G} = α_{C,ECT}-α_{O,MR}.

15. Verfahren nach Anspruch 8, **dadurch gekennzeichnet,**
**dass** mittels elektrischer Kapazitätstomographie der Wasseranteil α_{W} und der Kohlenwasserstoffanteil α_{C}, der sich additiv aus dem Ölanteil α_{O} und dem Gasanteil α_{G} gemäß α_{C} = α_{O}+α_{G} ergibt, gemessen werden, und der Wasseranteil α_{W} und der Kohlenwasserstoffanteil α_{C} aus der Verteilung der Permittivitäten oder aus der Verteilung der Leitfähigkeit des Mediums bestimmt werden,
**dass** durch das Messverfahren der Vormagnetisierungskontrastmessung das Verhältnis von Ölanteil α_{O} zu Wasseranteil α_{W} (OWR = α_{O}/α_{W}) bestimmt wird, wobei der Vormagnetisierungskontrast durch Variation der Länge der Vormagnetisierungsstrecke oder durch Variation der Messpositionen realisiert wird, und
**dass** mit den gemessenen Werten zunächst der Ölanteil α_{O} berechnet wird, indem der mittels elektrischer Kapazitätstomographie gemessene Wasseranteil α_{W} mit dem durch das Messverfahren der Vormagnetisierungskontrastmessung bestimmten OWR multipliziert wird, α_{O} = OWR · α_{W,ECT}, und anschließend der Gasanteil α_{G} bestimmt wird, indem der zuvor bestimmte Ölanteil α_{O} von dem mittels elektrischer Kapazitätstomographie gemessenen Kohlenwasserstoffanteil α_{C} subtrahiert wird, α_{G}=α_{C,ECT} - α_{O}.

16. Verfahren nach Anspruch 14 oder 15, **dadurch gekennzeichnet,**
**dass** die mittlere Leitfähigkeit des Mediums aus den mittels elektrischer Kapazitätstomographie gemessenen Werten bestimmt wird,
**dass** die durch die mittlere Leitfähigkeit des Mediums und/oder mindestens einer leitfähigen Phase eines mehrphasigen Mediums bedingte zusätzliche Last in dem RF-Resonator-Kreis des Magnetresonanzmessgerätes bestimmt wird und
**dass** die zur Anregung der Kernspins in das Medium eingespeiste RF-Leistung derart erhöht wird, dass der Einfluss der durch die mittlere Leitfähigkeit zusätzlich auftretenden Last auf die Anregung der Kernspins kompensiert wird.

17. Verfahren nach Anspruch 16, **dadurch gekennzeichnet,**
**dass** mittels elektrischer Kapazitätstomographie eine Leitfähigkeitskarte des Mediums über den Rohrquerschnitt erstellt wird, dass aus der Leitfähigkeitskarte die mittlere Leitfähigkeit des Mediums berechnet wird, und dass zusätzlich mit der Leitfähigkeitskarte die lokalen Abweichungen der Leitfähigkeit von der mittleren Leitfähigkeit des Mediums bestimmt werden,
**dass** die durch die mittlere Leitfähigkeit des Mediums bestimmte zusätzliche Last in dem RF-Resonator-Kreis des Magnetresonanzmessgerätes bestimmt wird und dass zusätzlich die lokalen aufgrund der Abweichungen der lokalen Leitfähigkeiten von der mittleren Leitfähigkeit auftretenden Dämpfungen des RF-Feldes bestimmt werden und
**dass** die zur Anregung der Kernspins in das Medium eingespeiste RF-Leistung derart erhöht wird, dass der Einfluss der durch die mittlere Leitfähigkeit zusätzlich auftretenden Last auf die Anregung der Kernspins kompensiert wird und zusätzlich lokal RF-Leistung in das Medium eingespeist wird, derart dass der Einfluss der von der mittleren Leitfähigkeit abweichenden lokalen Leitfähigkeiten auf die Anregung der Kernspins kompensiert wird.

18. Verfahren nach Anspruch 10 oder 16, **dadurch gekennzeichnet, dass** aus der Leitfähigkeit des Mediums und/oder mindestens einer leitfähigen Phase eines mehrphasigen Mediums der Salzgehalt in dem Medium und/oder in mindestens einer leitfähigen Phase des mehrphasigen Mediums bestimmt wird.
